# EUROPEAN PATENT APPLICATION

(11) **EP 3 434 993 A1**
(43) Date of publication of application: **30.01.2019**
(21) Application number: 18178974.4
(22) Date of filing: 21.06.2018
(51) Int. Cl.: F24F 11/30, F24F 11/52, F24F 11/58, F24F 110/50

(54) **AIR QUALITY NOTIFICATION AND PROCESSING SYSTEM**

(30) Priority: 27.07.2017 TW 106125336
(71) Applicant: Microjet Technology Co., Ltd, Hsinchu (TW)
(72) Inventor: Mou, Hao-Jan, Hsinchu (TW); Hsueh, Ta-Wei, Hsinchu (TW); Mo, Li-Pang, Hsinchu (TW); Chen, Shih-Chang, Hsinchu (TW); Lin, Ching-Sung, Hsinchu (TW); Huang, Chi-Feng, Hsinchu (TW); Han, Yung-Lung, Hsinchu (TW)
(74) Representative: Uexküll & Stolberg

(57) **Abstract**

An air quality notification and processing system (1) includes at least one notification-processing-connection device (10) and an air quality air quality processing device (20). The notification-processing-connection device (10) includes a display module (11) and a first communication module (12). After the first communication module (12) receives an air quality information, the air quality information is transmitted to the display module (11) and displayed on the display module (11). The air quality processing device (20) includes a second communication module (21). The second communication module (21) is in communication with the first communication module (12) to receive the air quality information.

## Description

### FIELD OF THE INVENTION

The present disclosure relates to an air quality notification and processing system, and more particularly to an air quality notification and processing system for reporting the air quality and facilitating improving the air quality.

### BACKGROUND OF THE INVENTION

Nowadays, people pay much attention to the air quality in the environment. For example, it is important to monitor carbon monoxide, carbon dioxide, volatile organic compounds (VOC), PM2.5, nitric oxide, sulfur monoxide, and so on. The exposure of these substances in the environment will cause human health problems or even harm the life. Therefore, it is important for every country to improve the air quality.

Generally, it is feasible to use an environmental sensor to monitor the air quality in the environment. If the environmental sensor is capable of immediately providing people with the monitored information relating to the environment for caution, it may help people escape or prevent from the injuries and influence on human health caused by the exposure of the substances described above in the environment. In other words, the environmental sensor is suitably used for monitoring the ambient air in the environment.

Nowadays, a large-scale environmental monitoring base station is provided to monitor the ambient air quality. However, the large-scale environmental monitoring base station is only suitable for monitoring the ambient air quality near the environmental monitoring base station. If the large-scale environmental monitoring base station is used to monitor the air quality in a small area where human activities exist (e.g., the indoor air quality and the ambient air surrounding us), the monitoring result is usually not accurately and quickly acquired. Consequently, the air quality cannot be effectively monitored everywhere and at any time.

As mentioned above, the air quality cannot be measured and improved by the current air quality monitoring system everywhere and at any time. Moreover, even if the process of improving the air quality is performed, the current air quality monitoring system cannot immediately realize whether the improved air quality is acceptable. If the air quality has been improved but the improved air quality is not recognized, the process of improving the air quality has to be continuously performed. Under this circumstance, the process of improving the air quality is workless and consumes energy.

Therefore, there is a need of providing an air quality notification and processing system for monitoring the air quality in real time, improving the air quality according to the monitored data in a cloud database, increasing the monitoring accuracy, immediately monitoring the air quality everywhere and at any time, and enabling the air quality notification mechanism and the air quality processing mechanism.

### SUMMARY OF THE INVENTION

In accordance with an aspect of the present disclosure, an air quality notification and processing system is provided. The air quality notification and processing system includes at least one notification-processing-connection device and at least one air quality processing device. The notification-processing-connection device includes a display module and a first communication module. The first communication module receives an air quality information and transmits the air quality information to the display module. The display module displays the air quality information. The air quality processing device includes a second communication module. The second communication module is in communication with the first communication module to receive the air quality information.

The above contents of the present disclosure will become more readily apparent to those ordinarily skilled in the art after reviewing the following detailed description and accompanying drawings, in which:

### BRIEF DESCRIPTION OF THE DRAWINGS

FIG. 1 schematically illustrates the architecture of an air quality notification and processing system according to a first embodiment of the present disclosure;
FIG. 2 schematically illustrates the architecture of an air quality notification and processing system according to a second embodiment of the present disclosure;
FIG. 3 schematically illustrates the architecture of an air quality notification and processing system according to a third embodiment of the present disclosure;
FIG. 4 schematically illustrates the architecture of an air quality notification and processing system according to a fourth embodiment of the present disclosure;
FIG. 5A is a schematic exploded view illustrating a fluid actuating device used in the actuating and sensing module of the present disclosure;
FIG. 5B is a schematic exploded view illustrating the fluid actuating device of FIG. 5A and taken along another viewpoint;
FIG. 6 is a schematic cross-sectional view illustrating the piezoelectric actuator of the fluid actuating device as shown in FIGS. 5A and 5B;
FIG. 7 is a schematic cross-sectional view illustrating the fluid actuating device as shown in FIGS. 5A and 5B; and
FIGS. 8A to 8E schematically illustrate the actions of the fluid actuating device of the actuating and sensing module according to the embodiment of the present disclosure.

### DETAILED DESCRIPTION OF THE PREFERRED EMBODIMENT

The present disclosure will now be described more specifically with reference to the following embodiments. It is to be noted that the following descriptions of preferred embodiments of this invention are presented herein for purpose of illustration and description only. It is not intended to be exhaustive or to be limited to the precise form disclosed.

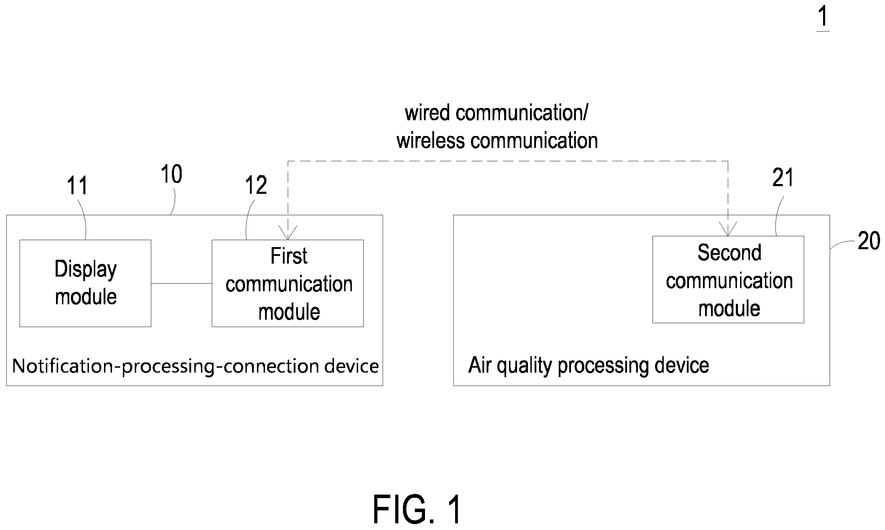

Please refer to FIG. 1. The present discourse provides an air quality notification and processing system 1 including at least one notification-processing-connection device 10, at least one display module 11, at least one first communication module 12, at least one air quality information, at least one air quality processing device 20 and at least one second communication module 21. The number of the display module 11, the first communication module 12, the air quality information and the second communication module 21 is exemplified by one for each in the following embodiments but not limited thereto. It is noted that each of the display module 11, the first communication module 12, the air quality information and the second communication module 21 can also be provided in plural numbers.

FIG. 1 schematically illustrates the architecture of an air quality notification and processing system according to a first embodiment of the present disclosure. As shown in FIG. 1, the air quality notification and processing system 1 of the present disclosure includes at least one notification-processing-connection device 10 and at least one air quality processing device 20. The notification-processing-connection device 10 includes a display module 11 and a first communication module 12. The first communication module 12 is electrically connected to the display module 11. The air quality processing device 20 includes a second communication module 21. The first communication module 12 and the second communication module 21 are in communication with each other.

Please refer to FIG. 1 again. The first communication module 12 of the notification-processing-connection device 10 is used for receiving and transmitting an air quality information. After the first communication module 12 receives the air quality information, the air quality information is transmitted to the display module 11. The air quality information is shown on the display module 11. Therefore, the user can be provided with the air quality information for consideration. Moreover, the air quality information is transmitted from the first communication module 12 to the second communication module 21 of the air quality processing device 20 through a wired communication path or a wireless communication path. According to the air quality information, the air quality processing device 20 is selectively enabled or disabled.

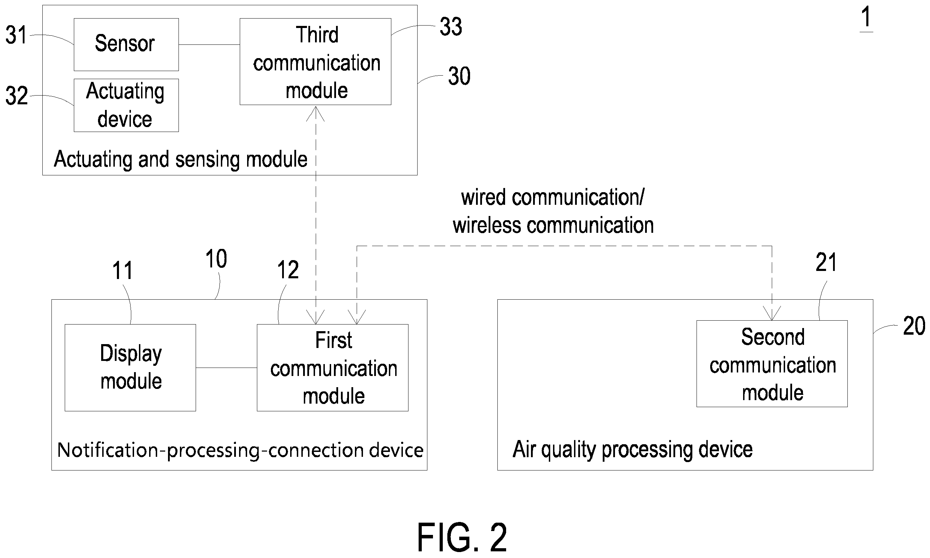

FIG. 2 schematically illustrates the architecture of an air quality notification and processing system according to a second embodiment of the present disclosure. As shown in FIG. 2, the structure of the air quality notification and processing system 1 of this embodiment is similar to that of the first embodiment. In comparison with the first embodiment, the air quality notification and processing system 1 of this embodiment further includes an actuating and sensing module 30. The actuating and sensing module 30 includes a sensor 31, an actuating device 32 and a third communication module 33. The sensor 31 is disposed adjacent to the actuating device 32 and electrically connected to the third communication module 33. When the actuating device 32 is enabled, the ambient air is inhaled into the interior of the actuating and sensing module 30 and transmitted to the sensor 31. After the ambient air is sensed by the sensor 31, an air quality information is generated. Then, the air quality information is transmitted to the third communication module 33. The third communication module 33 is in communication with the first communication module 12. Then, the air quality information is transmitted from the third communication module 33 to the first communication module 12 through a wired communication path or a wireless communication path. The air quality information is shown on the display module 11 of the notification-processing-connection device 10. Moreover, the air quality information is transmitted from the first communication module 12 to the second communication module 21 of the air quality processing device 20 through the wired communication path or the wireless communication path.

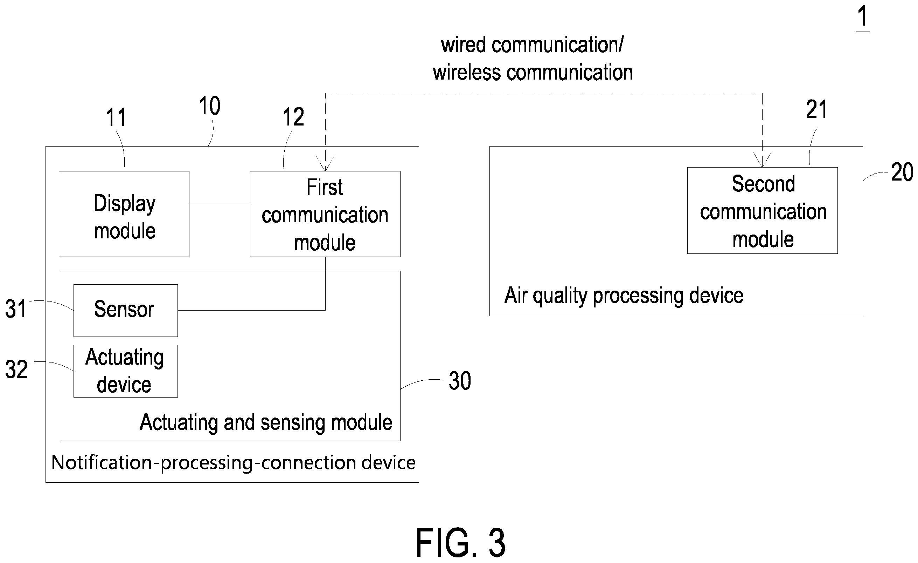

FIG. 3 schematically illustrates the architecture of an air quality notification and processing system according to a third embodiment of the present disclosure. As shown in FIG. 3, the structure of the air quality notification and processing system 1 of this embodiment is similar to that of the first embodiment. In comparison with the first embodiment, the notification-processing-connection device 10 of this embodiment further includes an actuating and sensing module 30. The actuating and sensing module 30 includes a sensor 31 and an actuating device 32. The sensor 31 is disposed adjacent to the actuating device 32 and electrically connected to the first communication module 12. When the actuating device 32 is enabled, the ambient air is inhaled into the interior of the actuating and sensing module 30 of the notification-processing-connection device 10 and transmitted to the sensor 31. After the ambient air is sensed by the sensor 31, an air quality information is generated. Then, the air quality information is transmitted to the first communication module 12. The air quality information is shown on the display module 11 of the notification-processing-connection device 10. Moreover, the air quality information is transmitted from the first communication module 12 to the second communication module 21 of the air quality processing device 20 through a wired communication path or a wireless communication path.

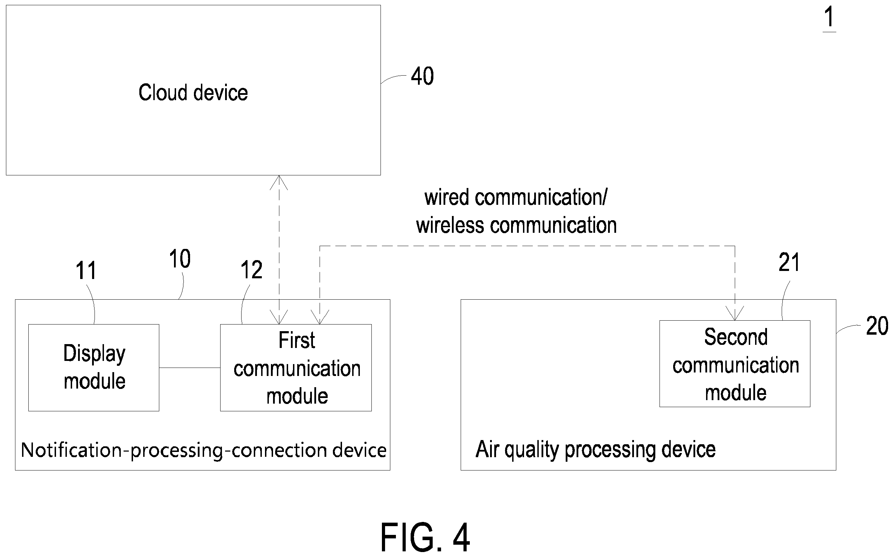

FIG. 4 schematically illustrates the architecture of an air quality notification and processing system according to a fourth embodiment of the present disclosure. As shown in FIG. 4, the structure of the air quality notification and processing system 1 of this embodiment is similar to that of the first embodiment. In comparison with the first embodiment, the air quality notification and processing system 1 of this embodiment further includes a cloud device 40. The notification-processing-connection device 10 is in communication with the cloud device 40 through the first communication module 12 using a wired communication path or a wireless communication path. Moreover, a position information of the notification-processing-connection device 10 is transmitted from the notification-processing-connection device 10 to the cloud device 40. According to the position information, an air quality information of the notification-processing-connection device 10 is transmitted from the cloud device 40 to the first communication module 12. Then, the air quality information is transmitted from the first communication module 12 to the display module 11 and shown on the display module 11. Therefore, the user can be provided with the air quality information for consideration.

For example, the air quality information includes the carbon monoxide concentration, the carbon dioxide concentration, the oxygen concentration, the PM2.5 particle concentration, the PM10 particle concentration, the ozone concentration, a volatile organic compound (VOC) concentration, the sulfur dioxide concentration, the nitrogen dioxide concentration, the humidity, the ammonia concentration, the methanol concentration, the alcohol concentration, or the combination thereof. Alternatively, the air quality information further includes the virus information, the bacterial information, the microbiological information, or the combination thereof.

After the notification-processing-connection device 10 of the air quality notification and processing system 1 receives the air quality information, the air quality information is shown on the display module 11. According to the air quality information, the user can realize whether the air quality is poor or the air quality is harmful to the human body. If the air quality is poor or the air quality is harmful to the human body, the air quality processing device 20 is enabled to improve the air quality.

An example of the air quality processing device 20 includes but is not limited to an air cleaner, a dehumidifier, a ventilating fan, an electric door, an electric window, an automatic cleaning robot or an air conditioner. After the air quality processing device 20 is enabled, the air quality processing device 20 can immediately improve the quality of the ambient air. After the quality of the ambient air is improved and the air quality information from the notification-processing-connection device 10 is received by the air quality processing device 20, the air quality processing device 20 is disabled. Consequently, the power-saving efficacy is enhanced.

When the air quality information is received by the second communication module 21 of the air quality processing device 20, the air quality processing device 20 performs an air quality processing mechanism. The air quality processing device 20 includes at least one intelligent home appliance. While the air quality processing mechanism is performed, the at least one intelligent home appliance is enabled. Preferably but not exclusively, the intelligent home appliance is an air cleaner, a dehumidifier, a ventilating fan, an electric door, an electric window, an automatic cleaning robot or an air conditioner. In some embodiments, a plurality of intelligent home appliances may be operated to improve the air quality. For example, after the air quality processing mechanism is enabled, the electric door is closed, the electric window is closed and the air cleaner is turned on. In this way, problems incurred by the PM2.5 particle concentration and the PM10 particle concentration can be improved.

In an embodiment, the first communication module 12 of the notification-processing-connection device 10 and the second communication module 21 of the air quality processing device 20 belong to the same kind of communication module. For example, the first communication module 12 and the second communication module 21 may be wireless transmission modules. The wireless communication module performs a wireless communication process (for establishing wireless communication path) by using a Zigbee communication technology, a Z-wave communication technology, an RF communication technology, a Bluetooth communication technology, a Wi-Fi communication technology or an EnOcean communication technology. Alternatively, the first communication module 12 and the second communication module 21 may also be wired transmission modules. For example, the wired communication module has a USB port, an RS485 communication port, an RS232 communication port, a Modbus communication port or a KNX communication port for performing a wired communication process (for establishing wired communication path).

After the notification-processing-connection device 10 receives the air quality information, a notification processing mechanism is enabled to notify the user to wear a mask or provide the user with an instant air quality map for consideration for evacuation or the like.

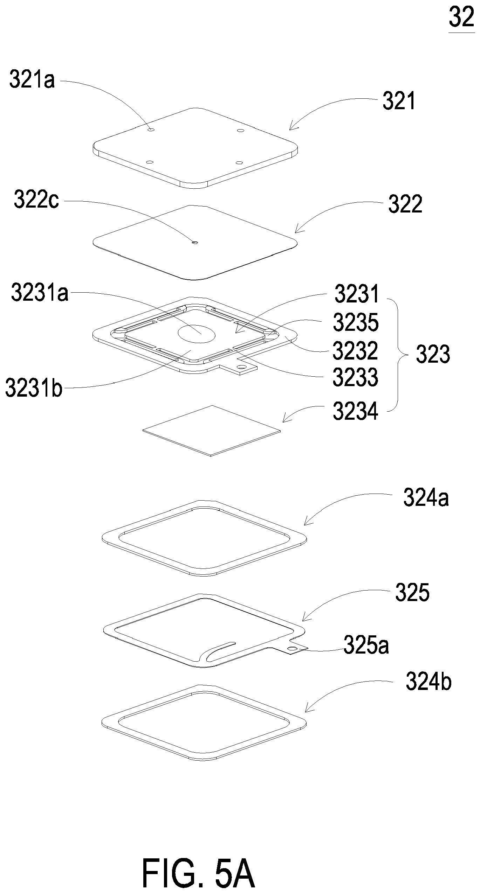

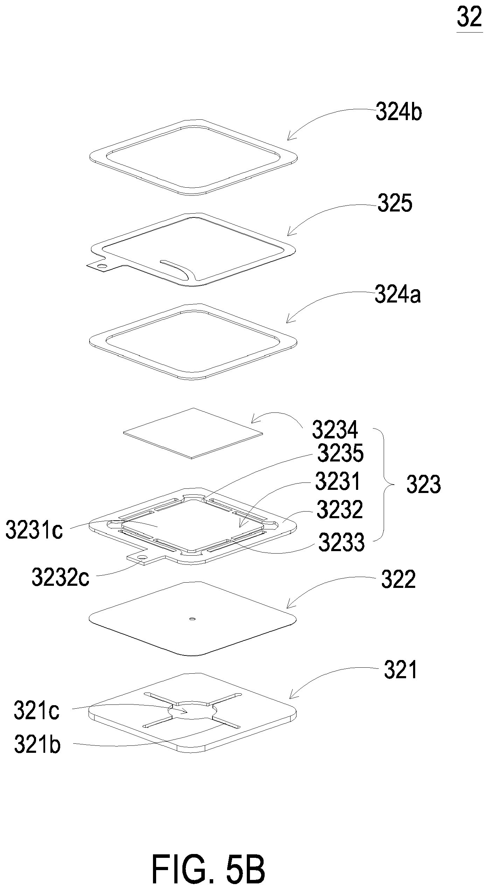

Please refer to FIGS. 5A and 5B. In an embodiment, the actuating device 32 is a fluid actuating device. The fluid actuating device 32 may be a driving structure of a piezoelectric pump or a driving structure of a micro-electro-mechanical system (MEMS) pump. Hereinafter, the actions of the fluid actuating device 32 of a piezoelectric pump will be described as follows.

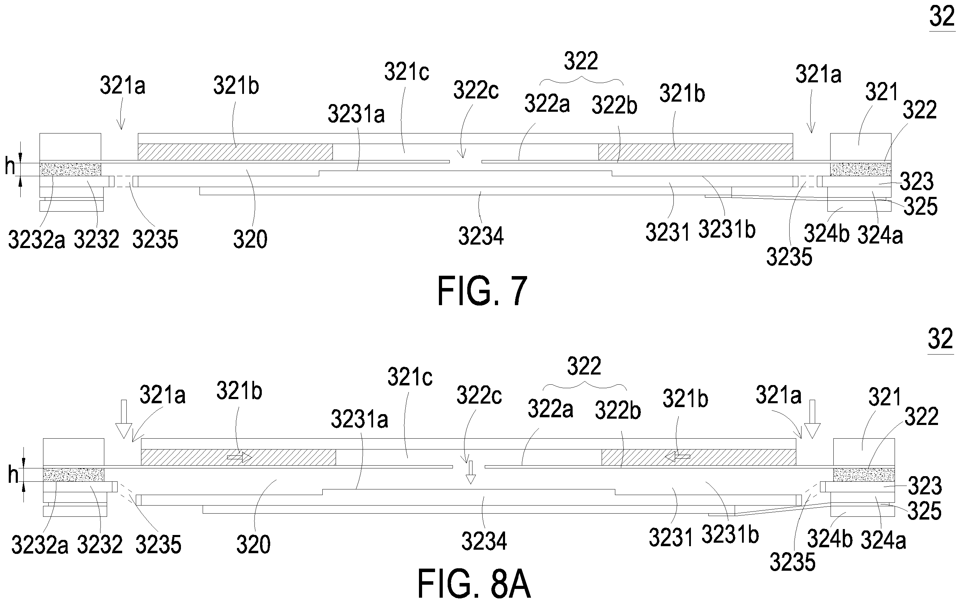

Referring to FIGS. 5A and 5B again. The fluid actuating device 32 includes a fluid inlet plate 321, a resonance plate 322, a piezoelectric actuator 323, a first insulation plate 324a, a conducting plate 325 and a second insulation plate 324b. The piezoelectric actuator 323 is aligned with the resonance plate 322. The fluid inlet plate 321, the resonance plate 322, the piezoelectric actuator 323, the first insulation plate 324a, the conducting plate 325 and the second insulation plate 324b are stacked on each other sequentially. After the above components are combined together, the cross-sectional view of the resulting structure of the fluid actuating device 32 is shown in FIG. 7.

The fluid inlet plate 321 includes at least one inlet 321a. Preferably but not exclusively, the fluid inlet plate 321 includes four inlets 321a. The inlets 321a run through the fluid inlet plate 321. In response to the action of the atmospheric pressure, the fluid can be introduced into the fluid actuating device 32 through the at least one inlet 321a. Moreover, at least one convergence channel 321b is formed on a first surface of the fluid inlet plate 321, and is in communication with the at least one inlet 321a on a second surface of the fluid inlet plate 321. Moreover, a central cavity 321c is located at the intersection of the convergence channels 321b. The central cavity 321c is in communication with the at least one convergence channel 321b, such that the fluid from the at least one inlet 131a would be introduced into the at least one convergence channel 321b and is guided to the central cavity 321c. In this embodiment, the at least one inlet 321a, the at least one convergence channel 321b and the central cavity 321c of the fluid inlet plate 321 are integrally formed from a single structure. The central cavity 321c forms a convergence chamber for temporarily storing the fluid. In some embodiments, the fluid inlet plate 321 may be, for example, made of stainless steel. Moreover, the depth of the convergence chamber defined by the central cavity 321c may be equal to the depth of the at least one convergence channel 321b. The resonance plate 322 is made of a flexible material. The resonance plate 322 has a central aperture 322c aligned with the central cavity 321c of the fluid inlet plate 321 which allows the fluid to be transferred therethrough. In other embodiments, the resonance 132 may be, for example, made of copper.

The piezoelectric actuator 323 includes a suspension plate 3231, an outer frame 3232, at least one bracket 3233 and a piezoelectric plate 3234. The piezoelectric plate 3234 is attached on a first surface 3231c of the suspension plate 3231. In response to an applied voltage, the piezoelectric plate 3234 is subjected to a deformation. When the piezoelectric plate 3234 is subjected to the deformation, it facilitates a bending vibration of the suspension plate 3231. The at least one bracket 3233 is connected between the suspension plate 3231 and the outer frame 3232, while the two ends of the bracket 3233 are connected with the outer frame 3232 and the suspension plate 3231 respectively that the bracket 3233 can elastically support the suspension plate 3231. At least one vacant space 3235 is formed between the bracket 3233, the suspension plate 3231 and the outer frame 3232. The at least one vacant space 3235 is in communication with a fluid channel for allowing the fluid to go through. The type of the suspension plate 3231 and the outer frame 3232 and the type and the number of the at least one bracket 3233 may be varied according to the practical requirements. The outer frame 3232 is arranged around the suspension plate 3231. Moreover, a conducting pin 3232c is protruded outwardly from the outer frame 3232 so as to be electrically connected with an external circuit (not shown).

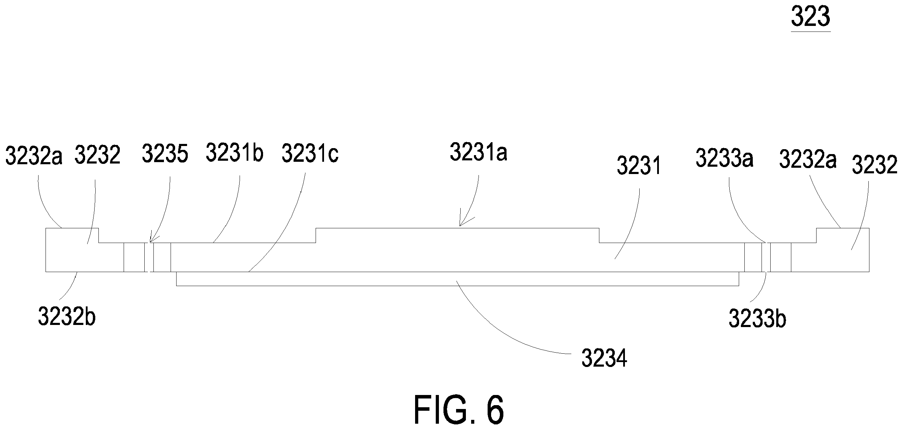

As shown in FIG. 6, the suspension plate 3231 has a bulge 3231a that makes the suspension plate 3231 a stepped structure. The bulge 3231a is formed on a second surface 3231b of the suspension plate 3231. The bulge 3231a may be a circular convex structure. A top surface of the bulge 3231a of the suspension plate 3231 is coplanar with a second surface 3232a of the outer frame 3232, while the second surface 3231b of the suspension plate 3231 is coplanar with a second surface 3233a of the bracket 3233. Moreover, there is a specific depth from the bulge 3231a of the suspension plate 3231 (or the second surface 3232a of the outer frame 3232) to the second surface 3231b of the suspension plate 3231 (or the second surface 3233a of the bracket 3233). A first surface 3231c of the suspension plate 3231, a first surface 3232b of the outer frame 3232 and a first surface 3233b of the bracket 3233 are coplanar with each other. The piezoelectric plate 3234 is attached on the first surface 3231c of the suspension plate 3231. In some other embodiments, the suspension plate 3231 may be a square plate structure with two flat surfaces but the type of the suspension plate 3231 may be varied according to the practical requirements. In this embodiment, the suspension plate 3231, the at least one bracket 3233 and the outer frame 3232 may be integrally formed from a metal plate (e.g., a stainless steel plate). In an embodiment, the length of a side of the piezoelectric plate 3234 is smaller than the length of a side of the suspension plate 3231. In another embodiment, the length of a side of the piezoelectric plate 3234 is equal to the length of a side of the suspension plate 3231. Similarly, the piezoelectric plate 3234 is a square plate structure corresponding to the suspension plate 3231 in terms of the design.

In this embodiment, the first insulation plate 324a, the conducting plate 325 and the second insulation plate 324b of the fluid actuating device 32 are stacked on each other sequentially and located under the piezoelectric actuator 323, as shown in FIG. 5A. The profiles of the first insulation plate 324a, the conducting plate 325 and the second insulation plate 324b substantially match the profile of the outer frame 3232 of the piezoelectric actuator 323. In some embodiments, the first insulation plate 324a and the second insulation plate 324b may be made of an insulating material (e.g. a plastic material) for providing insulating efficacy. In other embodiments, the conducting plate 325 may be made of an electrically conductive material (e.g. a metallic material) for providing electrically conducting efficacy. In this embodiment, the conducting plate 325 may have a conducting pin 325a disposed thereon so as to be electrically connected with an external circuit (not shown).

Please refer to FIG. 7. In an embodiment, the fluid inlet plate 321, the resonance plate 322, the piezoelectric actuator 323, the first insulation plate 324a, the conducting plate 325 and the second insulation plate 324b of the fluid actuating device 32 are stacked on each other sequentially. Moreover, there is a gap h between the resonance plate 322 and the outer frame 3232 of the piezoelectric actuator 323. In this embodiment, the gap h between the resonance plate 322 and the outer frame 3232 of the piezoelectric actuator 323, may be filled with a filler (e.g. a conductive adhesive) so that a depth from the resonance plate 322 to the bulge 3231a of the suspension plate 3231 of the piezoelectric actuator 323 can be maintained. The gap h ensures the proper distance between the resonance plate 322 and the bulge 3231a of the suspension plate 3231 of the piezoelectric actuator 323, so that the fluid can be transferred quickly, the contact interference is reduced and the generated noise is largely reduced. In some embodiments, alternatively, the height of the outer frame 3232 of the piezoelectric actuator 323 is increased, so that a gap is formed between the resonance plate 322 and the piezoelectric actuator 323.

Please refer to FIG. 5A, FIG. 5B and FIG. 7. After the fluid inlet plate 321, the resonance plate 322 and the piezoelectric actuator 323 are combined together, a movable part 322a and a fixed part 322b of the resonance plate 322 are defined. The movable part 322a is around the central aperture 322c. A convergence chamber for converging the fluid is defined by the movable part 322a of the resonance plate 322 and the fluid inlet plate 321 collaboratively. Moreover, a first chamber 320 is formed between the resonance plate 322 and the piezoelectric actuator 323 for temporarily storing the fluid. Through the central aperture 321c of the resonance plate 322, the first chamber 320 is in communication with the central cavity 321c of the fluid inlet plate 321. The peripheral regions of the first chamber 320 are in communication with the fluid channel through the vacant space 3235 between the brackets 3233 of the piezoelectric actuator 323.

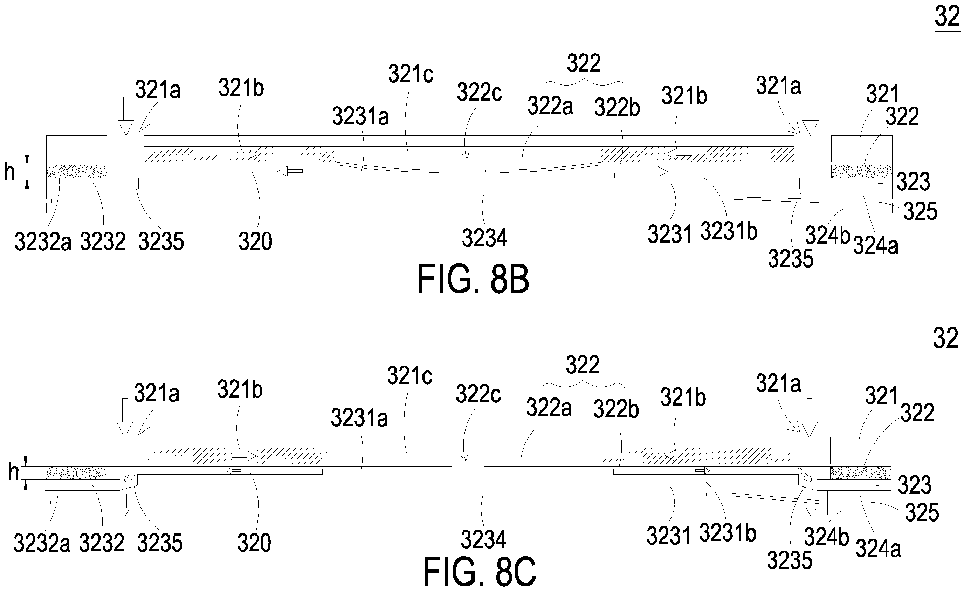

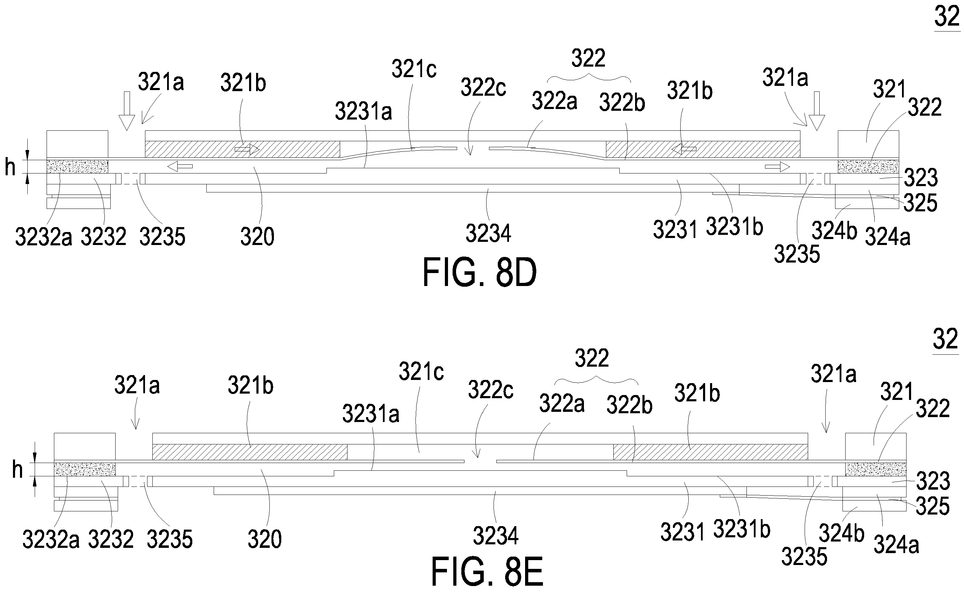

FIGS. 8A to 8E schematically illustrate the actions of the fluid actuating device of the actuating and sensing module according to the embodiment of the present disclosure. Please refer to FIG. 5A, FIG. 5B, FIG. 7 and FIGS. 8A to 8E. The actions of the fluid actuating device will be described as follows. When the fluid actuating device 32 is enabled, the piezoelectric actuator 323 vibrates along a vertical direction in a reciprocating manner by using the bracket 3233 as a fulcrum. Please refer to FIG. 8A, the piezoelectric actuator 323 vibrates downwardly in response to the applied voltage. Since the resonance plate 322 is light and thin, the resonance plate 322 vibrates along the vertical direction in the reciprocating manner in resonance with the piezoelectric actuator 323. More especially, a region of the resonance plate 322 spatially corresponding to the central cavity 321c of the fluid inlet plate 321 is also subjected to a bending deformation. The region of the resonance plate 322 corresponding to the central cavity 321c of the fluid inlet plate 321 is the movable part 322a of the resonance plate 322. When the piezoelectric actuator 323 deforms downwardly during vibration, the movable part 322a of the resonance plate 322 is subjected to the bending deformation because the movable part 322a of the resonance plate 322 is pushed by the fluid and vibrates in response to the piezoelectric actuator 323. In response to the downward deformation of the piezoelectric actuator 323 during vibration, the fluid is fed into the at least one inlet 321a of the fluid inlet plate 321. Then, the fluid is transferred to the central cavity 321c of the fluid inlet plate 321 through the at least one convergence channel 321b. Then, the fluid is transferred through the central aperture 322c of the resonance plate 322 spatially corresponding to the central cavity 321c, and introduced downwardly into the first chamber 320. As the piezoelectric actuator 323 is enabled, the resonance of the resonance plate 322 occurs. Consequently, the resonance plate 322 vibrates along the vertical direction in the reciprocating manner. As shown in FIG. 8B, during the vibration of the movable part 322a of the resonance plate 322 at this stage, the movable part 322a moves down to contact and attach on the bulge 3231a of the suspension plate 3231 of the piezoelectric actuator 323, and a distance from the fixed part 322b of the resonance plate 322 to a region of the suspension plate 3231 except the bulge 3231a remains the same. Owing to the deformation of the resonance plate 322 described above, a middle communication space of the first chamber 320 is closed, and the volume of the first chamber 320 is compressed. Under this circumstance, the pressure gradient occurs to push the fluid in the first chamber 320 moving toward peripheral regions of the first chamber 320 and flowing downwardly through the vacant space 3235 of the piezoelectric actuator 323. Referring to FIG. 8C, the movable part 322a of the resonance plate 322 returns to its original position when the piezoelectric actuator 323 deforms upwardly during vibration. Consequently, the volume of the first chamber 320 is continuously compressed to generate the pressure gradient which makes the fluid in the first chamber 320 continuously pushed toward peripheral regions. Meanwhile, the fluid is continuously fed into the at least one inlet 321a of the fluid inlet plate 321, and transferred to the central cavity 321c. Then, as shown in FIG. 8D, the resonance plate 322 moves upwardly, which is cause by the resonance of upward motion of the piezoelectric actuator 323. That is, the movable part 322a of the resonance plate 322 is also vibrated upwardly. Consequently, it decreases the current of the fluid from the at least one inlet 321a of the fluid inlet plate 321 into the central cavity 321c. At last, as shown in FIG. 8E, the movable part 322a of the resonance plate 322 has returned to its original position. As the embodiments described above, when the resonance plate 322 vibrates along the vertical direction in the reciprocating manner, the gap h between the resonance plate 322 and the piezoelectric actuator 323 is helpful to increase the maximum displacement along the vertical direction during the vibration. In other words, the configuration of the gap h between the resonance plate 322 and the piezoelectric actuator 323 can increase the amplitude of vibration of the resonance plate 322. Consequently, a pressure gradient is generated in the fluid channels of the fluid actuating device 32 to facilitate the fluid to flow at a high speed. Moreover, since there is an impedance difference between the feeding direction and the exiting direction, the fluid can be transmitted from the inlet side to the outlet side. Even if a gas pressure (which may impede the fluid flow) exists at the outlet side, the fluid actuating device 32 still has the capability of pushing the fluid to the fluid channel while achieving the silent efficacy. The steps of FIGS. 8A to 8E may be done repeatedly. Consequently, fluid circulation is generated in which the ambient fluid is transferred from the outside to the inside by the fluid actuating device 32.

From the above descriptions, the present disclosure provides an air quality notification and processing system. The air quality notification and processing system includes at least one notification-processing-connection device and at least one air quality processing device. The notification-processing-connection device is used for receiving the air quality information in real time. In case that the notification-processing-connection device is in communication with an actuating and sensing module, the air quality notification and processing system is capable of monitoring the air quality everywhere and at any time without being limited by the local monitoring station. In case that the notification-processing-connection device is in communication with the actuating and sensing module, the air quality notification and processing system is capable of determining whether any substance in air is harmful to human body, whereby various precautions can be taken against air pollution immediately if necessary. The at least one notification-processing-connection device is in communication with the at least one air quality processing device. If the air quality information indicates that the air quality is deteriorated, the notification-processing-connection device can immediately notify the air quality processing device to improve the air quality. Moreover, when the air quality processing device is enabled, the notification-processing-connection device monitors the air quality in real time and verifies whether the air quality is improved by the air quality processing device. In case that the air quality is effectively improved and the air quality is not harmful to the human body, the air quality processing device is disabled. Consequently, the power-saving efficacy is enhanced. As mentioned above, the air quality notification and processing system of the present disclosure is capable of monitoring the air quality everywhere and at any time and immediately reporting, processing and improving the air quality. If the air quality is acceptable, the air quality processing device is disabled so as to save the energy. In other words, the air quality notification and processing system of the present disclosure is industrially valuable.

While the invention has been described in terms of what is presently considered to be the most practical and preferred embodiments, it is to be understood that the invention needs not be limited to the disclosed embodiment. On the contrary, it is intended to cover various modifications and similar arrangements included within the spirit and scope of the appended claims which are to be accorded with the broadest interpretation so as to encompass all such modifications and similar structures.

## Claims

1. An air quality notification and processing system (1), comprising:
at least one notification-processing-connection device (10) comprising a display module (11) and a first communication module (12), wherein the first communication module (12) receives an air quality information and transmits the air quality information to the display module (11), and the display module (11) displays the air quality information; and
at least one air quality processing device (20) comprising a second communication module (21), wherein the second communication module (21) is in communication with the first communication module (12) to receive the air quality information.

2. The air quality notification and processing system (1) according to claim 1, wherein the air quality notification and processing system (1) further comprises at least one actuating and sensing module (30), and the at least one actuating and sensing module (30) comprises a sensor (31), an actuating device (32) and a third communication module (33), wherein the sensor (31) is disposed adjacent to the actuating device (32) and electrically connected to the third communication module (33), wherein when the actuating device (32) is enabled, an ambient air is inhaled into the actuating and sensing module (30) and transmitted to the sensor (31), wherein after the ambient air is sensed by the sensor (31) which generates the air quality information, the air quality information is transmitted to the first communication module (12) of the notification-processing-connection device (10) through the third communication module (33).

3. The air quality notification and processing system (1) according to claim 2, wherein the third communication module (33) is a wireless transmission module.

4. The air quality notification and processing system (1) according to claim 2, wherein the third communication module (33) is a wired transmission module.

5. The air quality notification and processing system (1) according to claim 1, wherein the at least one notification-processing-connection device (10) further comprises at least one actuating and sensing module (30), and the at least one actuating and sensing module (30) comprises a sensor (31) and an actuating device (32), wherein the sensor (31) is disposed adjacent to the actuating device (32) and electrically connected to the first communication module (12), wherein when the actuating device (32) is enabled, an ambient air is inhaled into the actuating and sensing module (30) and transmitted to the sensor (31), wherein after the ambient air is sensed by the sensor (31) which generates the air quality information, the air quality information is displayed on the display module (11) and transmitted to the first communication module (12).

6. The air quality notification and processing system (1) according to claim 1, wherein the air quality notification and processing system (1) further comprises a cloud device (40), and the air quality information is transmitted from the cloud device (40) to the first communication module (12) of the notification-processing-connection device (10).

7. The air quality notification and processing system (1) according to claim 1, wherein the air quality processing device (20) is one selected from the group consisting of an air cleaner, a dehumidifier, a ventilating fan, an electric door, an electric window, an automatic cleaning robot and an air conditioner.

8. The air quality notification and processing system (1) according to claim 1, wherein the air quality processing device (20) comprises at least one intelligent home appliance, wherein the air quality processing device (20) performs an air quality processing mechanism to operate the intelligent home appliance.

9. The air quality notification and processing system (1) according to claim 8, wherein the at least one intelligent home appliance is at least one selected from the group consisting of an air cleaner, a dehumidifier, a ventilating fan, an electric door, an electric window, an automatic cleaning robot and an air conditioner.

10. The air quality notification and processing system (1) according to claim 1, wherein the air quality information is at least one selected from the group consisting of a carbon monoxide concentration, a carbon dioxide concentration, an oxygen concentration, a PM2.5 particle concentration, a PM10 particle concentration, an ozone concentration, a volatile organic compound (VOC) concentration, a sulfur dioxide concentration, a nitrogen dioxide concentration, a humidity, an ammonia concentration, a methanol concentration, and an alcohol concentration.

11. The air quality notification and processing system (1) according to claim 1, wherein the first communication module (12) and the second communication module (21) are wired communication modules.

12. The air quality notification and processing system (1) according to claim 1, wherein the first communication module (12) and the second communication module (21) are wireless communication modules.

13. The air quality notification and processing system (1) according to claim 12, wherein the wireless communication modules are Bluetooth communication modules.

14. The air quality notification and processing system (1) according to claim 12, wherein the wireless communication modules are Wi-Fi communication modules.

15. An air quality notification and processing system (1), comprising:
at least one notification-processing-connection device (10) comprising at least one display module (11) and at least one first communication module (12), wherein the first communication module (12) receives at least one air quality information and transmits the air quality information to the display module (11), and the display module (11) displays the air quality information; and
at least one air quality processing device (20) comprising at least one second communication module (21), wherein the second communication module (21) is in communication with the first communication module (12) to receive the air quality information.
